# EUROPEAN PATENT APPLICATION

(11) **EP 1 936 357 A1**
(43) Date of publication of application: **25.06.2008**
(21) Application number: 07024245.8
(22) Date of filing: 13.12.2007
(51) Int. Cl.: G01N 21/35, H03F 3/45

(54) **Method of processing an analog sensor signal in a gas sensor arrangement and measured value processing device**

(30) Priority: 18.12.2006 DE 102006059652
(71) Applicant: Tyco Electronics Raychem GmbH, 85521 Ottobrunn (DE)
(72) Inventor: Minuth, Rudi, 85354 Freising (DE); Straub, Kuno, 85356 Freising (DE)
(74) Representative: Grünecker, Kinkeldey, Stockmair & Schwanhäusser Anwaltssozietät

(57) **Abstract**

The present invention relates to a method of processing an analog sensor signal, the analog sensor signal being amplified (502) by an operational amplifier (402). The amplified analog sensor signal is measured (504) and compared with a threshold value (506). A DC voltage is generated (508) as a function of a difference between the amplified analog sensor signal and the threshold value and a composite signal is formed (510) from the analog sensor signal and the DC voltage. The composite signal is amplified (512) and an output signal is output.

## Description

The present invention relates to a method of processing an analog sensor signal, the method using an operational amplifier.

In particular, the present invention relates to a method of this type for use in a gas sensor arrangement, to an associated gas sensor arrangement and a measured value processing device.

Known gas sensor arrangements comprise a radiation-emitting radiation source, a gas measuring chamber, which may be filled with a gas for measurement which contains at least one analyte to be measured, and at least one radiation-detecting detector device, which generates an output signal dependent on the presence and/or the concentration of the analyte. Such gas sensor arrangements are known for detecting the widest possible range of analytes, for example carbon dioxide or methane. Conventional gas sensors are based on the property of many polar gases to absorb radiation in the infrared range. In this case, the IR light is positioned to bring the molecules into excited states by inciting rotational and vibration oscillations through cooperation with the dipole moment of the polar molecule. In this way, the thermal energy of the IR light is transmitted to the gas and in the same way the intensity of an IR beam passing through the gas volume is reduced. In this respect, absorption occurs in accordance with the excitation states at a wavelength in each case characteristic of the relevant gas, in the case of CO₂ for example at 4.25 µm.

Carbon dioxide detection in particular is currently growing in importance in a plurality of fields of application. Thus, for example, in the automotive field carbon dioxide detection may be used to monitor the CO₂ content in the passenger compartment air for increasing the energy efficiency of the heating and air-conditioning systems, so that fresh air is supplied by means of corresponding fan vent actuation only when necessary, i.e. in the event of an elevated CO₂ concentration. Furthermore, modem automotive air-conditioning systems are also based on CO₂ as a coolant, such that CO₂ gas sensors may fulfil a monitoring function in the automotive field in connection with CO₂leakage in the event of faults. In the automotive field in particular, gas sensors have to satisfy the most stringent requirements for robustness, reliability and miniaturisability. For safety applications, moreover, the response time of the sensor must not exceed given limit values.

German patent application DE 102005032722 describes a gas sensor arrangement and a measurement method with early warning. In particular, this application relates to the radiation source, which emits radiation in the form of pulses. In addition, German patent application DE102006019705.4 relates to a method of processing time-discrete measured values, which may be described with regard to their time profile by means of a time function. The method according to this application uses a measured value filter to achieve a desired transient response.

In many gas sensor arrangements, "pyrosensors" are used as the detector device for evaluating the IR radiation. The analog sensor signal which is output by such a pyrosensor has a high offset voltage and only a small amplitude due to the nature of the measurement. For further processing and analysis of the signal it is helpful to remove this offset voltage, wherein, however, the amplitude of the analog sensor signal should be amplified.

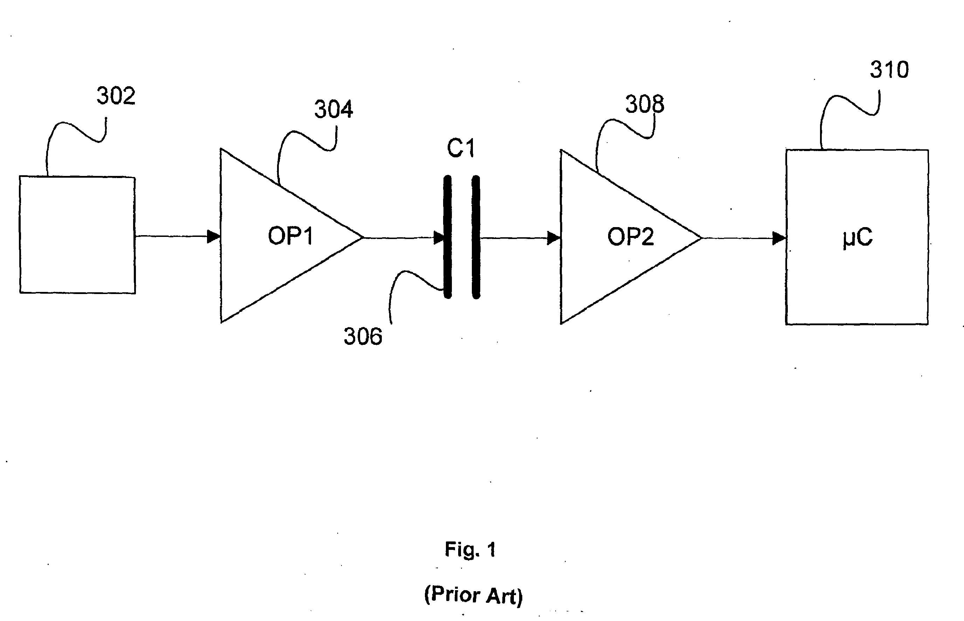

In known arrangements for example, as shown in Figure 1, the analog signal output by the sensor 302 is amplified in an operational amplifier 304. The offset voltage of this amplified signal is then filtered out by a capacitor 306, before the now corrected analog signal is amplified in a further operational amplifier 308. This amplified analog signal without offset voltage is then further processed in a microcontroller 310.

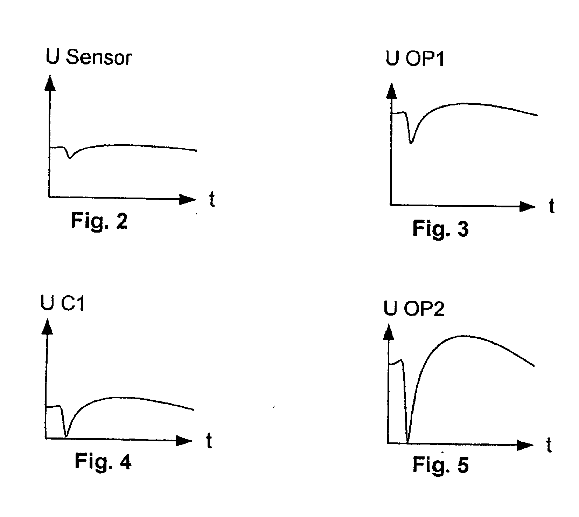

The four curves in Figures 2 to 5 show how the analog sensor signal is conditioned by means of the arrangement of Fig. 1. It is apparent that the signal output by the detector is of only very small amplitude in Figure 2 and that it has a large DC voltage content. The operational amplifier 304 amplifies the weak signal, but with it also the DC voltage content, such that the amplified signal may possibly leave the dynamic range of the subsequent evaluation. The capacitor 306 filters out the DC voltage content, see Figure 4, and the operational amplifier 308 amplifies this corrected signal to the extent that it may readily be measured, see Figure 5.

The capacitance of the coupling capacitor 306 is highly temperature-dependent, however, which may cause difficulties in the automotive field, since a given operating temperature cannot be guaranteed. In the temperature range from -40°C to +85°C the capacitor changes its high capacitance for example, and moreover this property is not stable in the long term. This coupling capacitor 306 is also unsuitable for easy integration into assemblies because of its size, and is therefore of complex and expensive design.

The object underlying the present invention therefore consists in providing an improved method of processing analog sensor signals, which enables temperature-independent, rapid and robust DC voltage suppression.

This object is achieved by the subject matter of the independent claims. Advantageous further developments of the present invention constitute the subject matter of the dependent claims.

The present invention is based on the concept that an improved temperature response arises upon amplification of the pure analog sensor signal without offset voltage, if the offset voltage can be removed without use of a capacitor. To this end, the output signal of the operational amplifier is fed back, to minimise offset.

According to the invention, simple, temperature-independent, linear control is achieved over the entire voltage range. It is advantageous that according to the invention a small number of microcontroller lines is necessary, for example just one signal line, so making possible a selection of more favorable, smaller processors. This also means a low current load and that independent control is possible by means of autonomous microcontroller peripherals, in particular by means of an integrated UART (Universal Asynchronous Receiver/Transmitter). This results in relieving of the microcontroller CPU (Central Processing Unit), which in turn means that simple regulating mechanisms may be used with low program memory consumption.

Use according to the invention of a pulse-width-modulated signal for controlling the DC voltage has the advantage of time optimisation due to the possibility of rapid range variation.

The solution according to the invention also has the advantage that, in the event of a sudden increase in gas concentration, more rapid processing of the analog sensor signal may be guaranteed.

The advantageous features of the measured value processing according to the invention may be exploited in particular in gas sensor arrangements which are used to detect carbon dioxide, for example in the automotive field, both for monitoring CO₂ escaping from leakage points and for checking the air quality in the passenger compartment. It goes without saying, however, that the principles according to the invention may also be used in connection with the detection of any other desired gases and are of significance for all sensors in which a measurement signal with a high DC voltage content needs to be evaluated.

The invention is explained more fully below with reference to the advantageous embodiments illustrated in the attached drawings. Similar or corresponding details of the subject matter according to the invention are provided with the same reference signs. In the drawings:
Fig. 1 is a circuit diagram for a known measured value processing device;
Fig. 2 shows a signal profile over time for the arrangement of Fig. 1;
Fig. 3 shows a signal profile over time for the arrangement of Fig. 1;
Fig. 4 shows a signal profile over time for the arrangement of Fig. 1;
Fig. 5 shows a signal profile over time for the arrangement of Fig. 1;
Fig. 6 is a schematic representation of a gas sensor unit according to the present invention;
Fig. 7 is a schematic representation of a measured value processing device according to the invention; and
Fig. 8 shows a flow chart of the method according to the invention.

The structure of the gas sensor arrangement according to the invention and the mode of operation of the method according to the invention for amplifying the analog sensor signal will be explained in greater detail below with reference to the Figures.

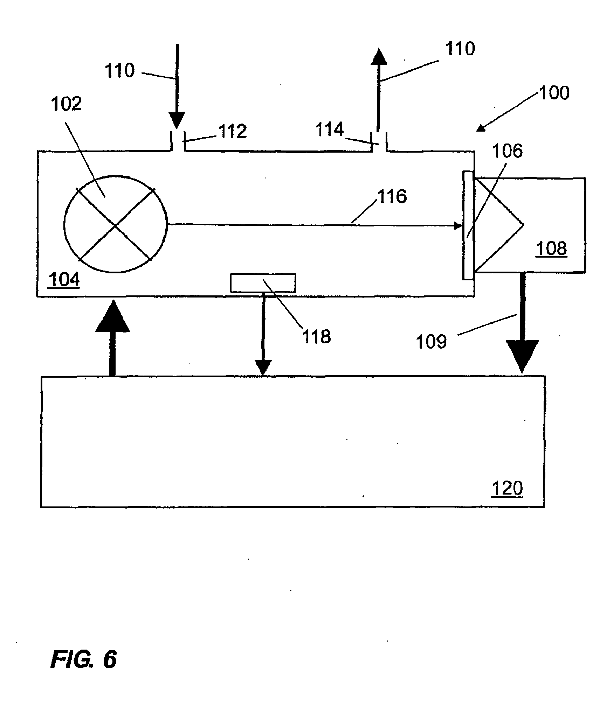

As shown in Fig. 6, a detector unit 108 detects radiant energy, which also depends on the concentration of the gas mixture. It goes without saying that the input signal for the detector unit 108 does not have specifically to be a gas concentration, but rather any desired sensor may be influenced in its time response in accordance with the principles of the present invention. The detector unit 108 supplies a time-discrete detector signal 109, which has an offset.

Fig. 6 shows an overview of a gas sensor arrangement which uses the filter unit according to the invention. As this Figure shows, the gas sensor arrangement 100 according to the invention comprises a radiation source 102, here a broadband infrared radiation source. In principle, the gas sensor arrangement 100 shown is a so-called NDIR (Non-Dispersive Infrared) sensor. In addition to the infrared radiation source 102, the essential components are the gas measuring chamber 104, a wavelength filter 106 and, as detector unit 108, an infrared detector. The temperature can be measured with a temperature sensor 118. The gas to be measured 110, which is to be checked for the gas component to be detected, is pumped into the gas measuring chamber 104 or diffused thereinto, as is symbolised by the in- and outlets 112, 114. The presence and/or concentration of the gas sought may, as explained above, be determined electro-optically via the absorption of a specific wavelength in the infrared range. Figure 2 shows the dynamic profile of the detector signal as a response to switching on and off of the lamp.

Here, the emitted infrared radiation 116 is passed through the gas measuring chamber 104 to the detector unit 108. An optical filter is arranged on the detector unit 108, which filter allows through only the wavelength range in which the gas molecules to be detected are absorbent. Other gas molecules normally absorb at other specific wavelengths and therefore have no influence on the amount of radiation which reaches the detector unit 108. Any suitable infrared detectors may be used as the detector unit and the one according to the invention may be adapted to a particular detector type.

For example, the detector may be a pyroelement, an infrared thermopile or a photodiode. The respectively suitable detector should be selected in accordance with the respective requirements. For instance, a photodiode offers the advantage that it represents a comparatively inexpensive component, while the thermocouple pile, as the thermopile detector is also known, offers the advantage of a particularly elevated and uniform absorption of the radiation in the selected range of the spectrum. Finally, pyroelectric sensors have the advantage of highly elevated sensitivity and miniaturised production.

The infrared signal is pulsed by the radiation source 102, in order to generate a dynamic signal, since the pyroelectric sensor only responds to variations. Thus, the measured values supplied by the detector unit are present in the form of time-discrete values, which, as indicated in Fig. 2, fulfil an exponential function.

A controller 120 on the one hand drives the radiation source 102 and on the other hand receives the detector signals from the detector unit 108 and further processes them according to the principles of the present invention. In particular, the controller comprises a filter unit 109, which performs conversion of the detector signal into an amplified output signal without offset.

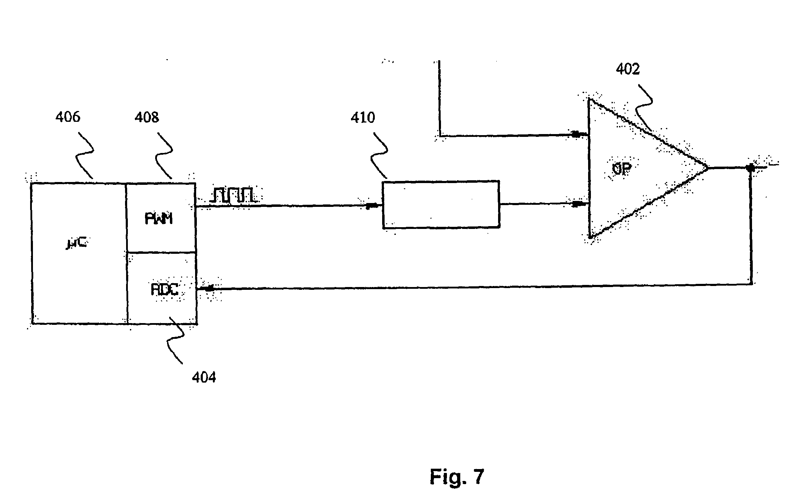

As shown in Fig. 7, the analog sensor signal is fed into an input of the operational amplifier 402. The output signal of the operational amplifier is connected to the analog-to-digital converter 404, which is controlled by the control device 406. The control device 406 also controls the pulse-width-modulated signal generator 408, which outputs a pulse-width-modulated signal. The filter 410 filters this pulse-width-modulated signal before it is fed into another input of the operational amplifier 402.

The mode of operation of the measured value processing device according to the invention is described below.

A microcontroller-controlled voltage is added to the analog sensor signal in the operational amplifier 402, and the difference is simultaneously amplified to generate an output signal. The microcontroller 406 measures the output signal, for which purpose an analog-to-digital converter 404 is used, which converts the amplified analog output signal from the operational amplifier 402 into a digital input signal for the microcontroller 406. The microcontroller 406 produces a signal correction by means of the digital input signal and the predetermined threshold value.

This threshold value may be determined by the operating voltage of the microcontroller 406, but other factors may also come into play. If the output signal of the operational amplifier 402 is not in the desired range, countermeasures are then taken using a pulse-width-modulated signal smoothed by a filter 410, and thus a DC voltage, in order to suppress the offset signal. The result is amplification of the pure analog signal without offset voltage, as may be seen in the time profile in Fig. 5. In this way, the amplified analog sensor signal is brought into the active measurement range.

In the invention the analog sensor signal corresponds to the signal in Fig. 3, and the PWM signal is a pure square-wave signal with different widths and intervals (controlled by the microcontroller 406). The subsequent filter 410 converts the square-wave signal into a constant voltage, which is proportional to the area of the incoming square wave. Thus, if the pulse/width ratio of the square-wave signal is varied, the area and thus the constant voltage downstream of the filter 410 are varied.

The microcontroller 406 may, for example, detect in the time profile that the voltage of the output signal is reaching its maximum operating voltage. The signal arrives at the analog-to-digital converter 404 internal to the microcontroller 406, which converter only admits signals up to the reference voltage. Then the pulse-width-modulated signal is varied in such a way (smaller pulse-width ratio) that a smaller constant voltage is generated downstream of the filter 410. The operational amplifier 402 forms the sum of the two signals, which has then in this case become smaller, and simultaneously amplifies the result in order to achieve better signal evaluation. The signal output of the operational amplifier 402 is then comparable with the signal "U OP2" in Fig. 5.

During measured value recording over the time profile the pulse-width-modulated signal (pulse-width ratio) has naturally to be constant. After measurement, adjustment may optionally be performed (pulse-width ratio is varied), and then new measurement is started.

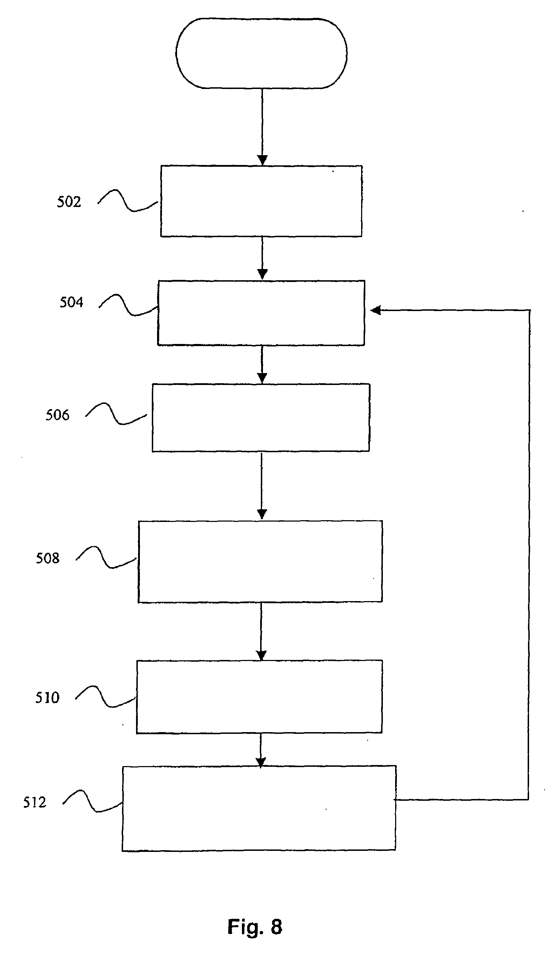

Fig. 8 illustrates the method according to the invention in the form of a flow chart. The output signal of the pyrosensor is amplified as an analog sensor signal in step 502 by the operational amplifier. The offset of the analog sensor signal is therefore also amplified in this step. This amplified analog sensor signal is measured in step 504 and compared with a threshold value in step 506. This threshold value is a predetermined voltage, which depends inter alia on the maximum operating voltage of the control device. The microcontroller controls generation of a pulse-width-modulated signal to the effect that, after filtering of the pulse-width-modulated signal, a DC voltage is obtained (step 508), the value of which depends on the difference between the output signal of the operational amplifier, and thus the amplified analog sensor signal, and the given threshold value.

The operational amplifier then adds this direct voltage to the analog sensor signal in step 510 and amplifies this composite signal in step 512, in order then to output an output signal.

The greater the difference between the amplified analog sensor signal and the threshold value in step 508, the smaller should be the value of the DC voltage to be added, so that the output signal of the operational amplifier has only a small offset. Conversely, the DC voltage to be added should have a higher value if there is a big difference between the amplified analog sensor signal and the threshold value. The reason for this is that the output signal of the operational amplifier should be amplified to the greatest possible extent without the operating voltage of the microcontroller being exceeded. The available amplitude should thus be utilised. If the amplified analog sensor signal, i.e. the amplified output signal of the pyrosensor, is already close to the operating voltage of the microcontroller, then not much more may be added thereto, but if the amplified output signal of the pyrosensor is weak, a larger DC voltage may be added thereto before the maximum operating voltage of the microcontroller is achieved. It is here assumed that the reference variable is always the amplified analog sensor signal, i.e. the output signal of the operational amplifier.

For most applications in gas sensors it is not only the final value of the signal but rather in particular the slope of the signal which is the variable of interest.

This will be explained in greater detail with reference to Figures 2 to 5. The output signal of the pyrosensor is given in Figure 2. As is clarified in Fig. 3, this signal is amplified, such that the amplitude of the signal is amplified and also the offset is enlarged. This signal in Figure 3 is the amplified analog sensor signal. The output signal of the operational amplifier is then illustrated in Figure 5, from which it is clearly possible to see that the offset has been reduced and the amplitude of the signal has been amplified. This effect cannot for example be achieved with an Automatic Gain Control (AGC), since the offset would always be amplified therewith.

With the measured value processing according to the invention, it is possible in particular, in connection with gas sensors, to achieve a more rapid response by the entire sensor system to suddenly increased gas concentration values. Although the above description refers all the time to the particular case of an NDIR CO₂ sensor, it is clear that the present invention may be adapted to all sensor systems in which an analog sensor signal with offset is present.

## Claims

1. A method of processing an analog sensor signal, the method comprising the following steps:
amplification of the analog sensor signal by an operational amplifier;
measurement of the amplified analog sensor signal;
comparison of the amplified analog sensor signal with a threshold value;
generation of a DC voltage as a function of a difference between the amplified analog sensor signal and the threshold value;
formation of a composite signal from the analog sensor signal and the DC voltage; and
amplification of the composite signal and outputting of an output signal.

2. A method of processing an analog sensor signal according to claim 1, the step of generating a DC voltage comprising:
generation of a first DC voltage value if the threshold value is not reached, or generation of a second, reduced DC voltage value if the threshold value is exceeded.

3. A method of processing an analog sensor signal according to claim 1 or claim 2, the step of generating the DC voltage comprising:
adjustment of a pulse-duty factor of a pulse-width-modulated signal as a function of the difference and filtering of the pulse-width-modulated signal for conversion into the DC voltage.

4. A method of processing an analog sensor signal according to claim 2, the DC voltage being proportional to an integral of a voltage over time of the pulse-width-modulated signal.

5. A method of processing an analog sensor signal according to at least one of the preceding claims, the step of measuring the amplified analog sensor signal comprising:
conversion of the amplified analog sensor signal into a digital signal and evaluation of the digital signal using a control device.

6. A method according to claim 4, the threshold value being a maximum operating voltage of the control device.

7. A method of processing an analog sensor signal according to at least one of the preceding claims, the analog sensor signal being generated by a pyrosensor.

8. A method according to at least one of the preceding claims, the analog sensor signal indicating the presence and/or the concentration of a polar gas, preferably carbon dioxide.

9. A measured value processing device for processing an analog sensor signal, the measured value processing device comprising:
an operational amplifier (402) for amplifying the analog sensor signal;
a control device (406) for measuring the amplified analog sensor signal and for comparing the amplified analog sensor signal with a threshold value;
a DC voltage being generated as a function of a difference between the amplified analog sensor signal and the threshold value; and
the operational amplifier (402) amplifying a sum of the analog sensor signal and the DC voltage and outputting an output signal.

10. A measured value processing device according to claim 9, further comprising:
an analog-to-digital converter (404) for converting the amplified analog sensor signal into a digital signal, the control device (406) evaluating the digital signal.

11. A measured value processing device according to claim 9 or claim 10, the control device (406) being adapted to adjust a pulse-duty factor of a pulse-modulated signal as a function of the difference and the measured value processing device further comprising:
a filter for filtering (410) the pulse-width-modulated signal for conversion into the DC voltage.

12. A gas sensor arrangement (100) having at least one radiation-emitting radiation source (102), a gas measuring chamber (104), which may be filled with a gas for measurement which contains at least one analyte to be measured, at least one radiation-detecting detector unit (108), which generates an analog sensor signal dependent on the presence and/or the concentration of the analyte, and having a measured value processing device for detecting the analog sensor signal and outputting an amplified output signal,
the measured value processing device being adapted to perform the method according to one of claims 1 to 8.
